(19) Europäisches Patentamt
European Patent Office
Office européen des brevets

(11) Veröffentlichungsnummer: **0 613 892 A1**

# (12) EUROPÄISCHE PATENTANMELDUNG

(21) Anmeldenummer: **94100394.9**

(51) Int. Cl.[5]: **C07D 295/20**

(22) Anmeldetag: **13.01.94**

(30) Priorität: **22.01.93 AT 94/93**

(43) Veröffentlichungstag der Anmeldung:
**07.09.94 Patentblatt 94/36**

(84) Benannte Vertragsstaaten:
**AT BE CH DE DK ES FR GB GR IT LI NL SE**

(71) Anmelder: **Chemie Linz GmbH**
**St.Peter-Strasse 25**
**A-4021 Linz (AT)**

(72) Erfinder: **Hackl, Kurt Alfred, Dipl.-Ing. Dr.**
**Anzengruberstrasse 3**
**A-4020 Linz (AT)**
Erfinder: **Rössler, Markus**
**Gabesstrasse 9**
**A-4030 Linz (AT)**
Erfinder: **Müllner, Martin, Dipl.-Ing. Dr.**
**Grabnerstrasse 33**
**A-4020 Linz (AT)**
Erfinder: **Stern, Gerhard, Dipl.-Ing. Dr.**
**Unterrudersbach 7**
**A-4180 Sonnberg (AT)**

(74) Vertreter: **Kunz, Ekkehard, Dr.**
**Chemie Linz GmbH**
**Abteilung Patente**
**St. Peter-Str. 25**
**A-4021 Linz (AT)**

## (54) Verwendung und Verfahren zur Herstellung von N-cyclischen und N,N'-dicyclischen Harnstoffen.

(57) Verwendung von Harnstoffen, in denen zumindest eines der beiden Stickstoffatome des Harnstoffes Teil eines nicht aromatischen Ringes, der durch ein Sauerstoff-oder Schwefelatom durchbrochen sein kann, ist, als chemisches Lösungsmittel, sowie ein Verfahren zur Herstellung von tri- oder tetraalkylierten Harnstoffen oder Diharnstoffen, wobei zumindest eines der Stickstoffatome des Harnstoffes Teil eines nicht aromatischen Ringes, der durch ein Sauerstoff- oder Schwefelatom durchbrochen sein kann, ist, durch Dialkylierung der $-NH_2$-Gruppe eines Harnstoffes mit einem Alkylen-oder Alkylenarylenalkylendihalogenid, -disulfonat oder -dihydrogensulfat in Gegenwart einer festen Base und eines Phasentransferkatalysators.

EP 0 613 892 A1

Chemische Reaktionen werden im allgemeinen in einem Lösungs- und Verdünnungsmittel durchgeführt, da das direkte Zusammenbringen von Reaktionspartnern normalerweise keine Kontrolle der Reaktionen erlaubt und da viele Reaktionen überhaupt nur ablaufen, wenn die Reaktionspartner in gelöstem Zustand vorliegen. Anforderungen, die an ein Lösungsmittel für chemische Reaktionen gestellt werden, sind hohe thermische Stabilität, gute Destillierbarkeit, Farblosigkeit, Ungiftigkeit, inertes Verhalten gegenüber den Reaktanten, Mischbarkeit mit anderen Lösungsmitteln und vor allem ein gutes Lösungsvermögen sowohl für polare oder hydrophile, als auch für unpolare oder hydrophobe Verbindungen. Es gibt aber kaum Lösungsmittel, die alle diese Anforderungen erfüllen.

Aus E. Müller, Houben-Weyl, 4. Auflage, Band E4, Seite 335 ist bekannt, daß tetraalkylierte Harnstoffe, wie Tetramethyl- oder Tetraethylharnstoff oder N,N'-überbrückte Harnstoffe, wie 1,3-Dimethyl-2-oxo-imidazolidin, 1,3-Dimethyl-2-imidazolidinon oder 1,3-Dimethyl-2-oxo-hexahydropyrimidin aufgrund ihrer günstigen Eigenschaften als aprotische Lösungsmittel für technische Zwecke genutzt wurden. Es wurden nun unerwarteterweise gefunden, daß chemisch anders geartete Harnstoffe, nämlich tetraalkylierte, N-cyclische oder N,N'-dicyclische Harnstoffe, insbesonders N-substituierte 1-Pyrrolidin-, 1-Piperidin- oder 1-Morpholincarbonsäureamidderivate im allgemeinen flüssig vorliegen, eine hohe thermische Stabilität aufweisen, gut destillierbar, farblos, ungiftig und gegenüber fuktionellen Gruppen völlig inert sind und für verschiedenste Reaktionen und Anwendungen universelle und ausgezeichnete Lösungsmittel sowohl für polare oder hydrophile als auch für unpolare oder hydrophobe Verbindungen darstellen. Unerwarteterweise hat sich gezeigt, daß solche Harnstoffe trotz des hohen hydrophoben Anteils außer mit anderen organischen Lösungsmitteln auch mit Wasser mischbar sind. Außerdem sind diese Harnstoffe ausgezeichnet befähigt, stark polare oder ionische Verbindungen, beispielsweise Salze, zu lösen. Da organische Lösungsmittel zumeist stark polare oder ionische Verbindungen nicht zu lösen vermögen, kann dieser überraschende Effekt beispielsweise für Reaktionen mit Salzen im nicht wäßrigen Medium genutzt werden.

Gegenstand der Erfindung ist daher die Verwendung eines Harnstoffes der allgemeinen Formel

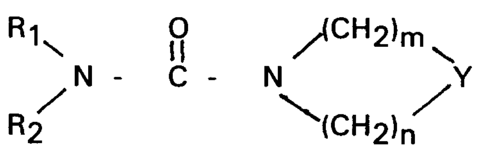

I

in der $R_1$ und $R_2$ unabhängig voneinander geradkettige, verzweigte oder cyclische, unsubstituierte oder durch Fluoratome, Nitrogruppen, Alkenyl-, Alkyliden-, Aryl-, Alkoxy- oder Phenoxygruppen substituierte Alkyl- oder Aralkylgruppen oder gemeinsam mit dem Stickstoffatom einen 5- oder 6-gliedrigen, nicht aromatischen Ring, der durch ein Sauerstoff- oder Schwefelatom durchbrochen sein kann, Y eine Methylengruppe, ein Sauerstoff- oder Schwefelatom und n und m unabhängig voneinander die Zahlen 1 bis 3, wobei n plus m die Zahlen 3 oder 4 sind, bedeuten, als chemisches Lösungsmittel.

Unter Alkylgruppen sind dabei Alkylgruppen mit 1 bis 22, bevorzugt mit 1 bis 10 C-Atomen, besonders bevorzugt mit 1 bis 8, ganz besonders bevorzugt mit 1 bis 6 C-Atomen, zu verstehen, wie z.B. Ethyl-, Propyl-, iso-Propyl-, tert.Butyl-, iso-Pentyl-, Methylcyclopentyl-, Cyclohexyl-, 2-Ethylhexyl-, Octyl-, Decyl-, Dodecyl-, Hexadecyl-, Octadecylgruppen.

Die Alkylgruppen können unsubstituiert oder durch Fluoratome, Nitrogruppen, Alkenyl-oder Alkylidengruppen mit 2 bis 6 C-Atomen, Arylgruppen, Alkoxygruppen mit 1 bis 5 C-Atomen, z.B. Methoxy-, Ethoxy-, iso-Propoxy-, Butoxygruppen oder Phenoxygruppen substituiert sein. Bevorzugt sind die Alkylgruppen unsubstituiert. Arylgruppen sind unsubstituierte oder durch Fluoratome, Nitrogruppen, Alkylgruppen mit 1 bis 5 C-Atomen oder Alkoxygruppen mit 1 bis 5 C-Atomen substituierte Phenylgruppen.

Unter Aralkylgruppen sind Benzyl- oder Phenylethylgruppen, wobei die Phenylgruppen durch Alkylgruppen mit 1 bis 5 C-Atomen, z.B. Ethyl-, iso-Propyl-, iso-Pentylgruppen, Alkoxygruppen mit 1 bis 5 C-Atomen, z.B. Methoxy-, Ethoxy-, iso-Propoxy-, Butoxygruppen, Fluoratome oder Nitrogruppen substituiert sein können, zu verstehen.

$R_1$ und $R_2$ können auch gemeinsam mit dem Stickstoffatom einen 5- oder 6-gliedrigen, nicht aromatischen Ring bilden, der durch ein Sauerstoff- oder Schwefelatom durchbrochen sein kann, also z.B. einen Oxazolidin-, Pyrrolidin-, Piperidin-, Morpholin-, Thiomorpholin-, Thiazolidinring.

Bevorzugt bedeuten $R_1$ und $R_2$ unabhängig voneinander eine unsubstituierte, geradkettige oder verzweigte Alkylgruppe mit 1 bis 10 C-Atomen oder $R_1$ und $R_2$ bedeuten gemeinsam mit dem Stickstoffatom einen nicht aromatischen Ring, der durch ein Sauerstoff- oder Schwefelatom durchbrochen sein kann, bevorzugt den Pyrrolidin-, Piperidin- oder Morpholinring.

Y bedeutet eine Methylengruppe, ein Sauerstoff- oder ein Schwefelatom, bevorzugt eine Methylengruppe oder ein Sauerstoffatom und m und n unabhängig voneinander die Zahlen 1 bis 3, bevorzugt 1 bis 2, wobei die Summe aus m plus n die Zahlen 3 oder 4 bedeutet.

Verfahren zur Herstellung von Harnstoffen sind etwa in U.Petersen in E. Müllner, Houben Weyl, 4. Auflage, Bd. E4, Seiten 338ff geoffenbart. In DE-A1 32 00 648 ist beschrieben, daß Amidverbindungen, unter denen auch Harnstoff verstanden wird, durch gleichzeitiges Inkontaktbringen und Umsetzen einer stark basischen Substanz mit der Amidverbindung und einer halogensubstituierten Verbindung in einem aprotischen, polaren Verdünnungsmittel am Stickstoffatom bzw. an beiden Stickstoffatomen alkyliert werden kann, wenn die Reaktion begonnen wird, solange die basische Substanz in einem suspendierten Zustand vorliegt. Das Verfahren ist aber nur zur Herstellung von symmetrischen, gegebenenfalls substituierten, Methylharnstoffen ausgehend von Harnstoff und nicht zur Herstellung N-cyclischer oder N,N'-dicyclischer Harnstoffe geeignet.

In JP-B-4-8425 (Chemical Abstracts Band 112, 198399 j) ist den Beispielen 7 und 8 angegeben, daß Carbonylbispyrrolidin bzw. Carbonylbispiperidin durch Umsetzung von Harnstoff mit 1,4-Dibrombuton in Gegenwart von Kaliumhydroxid in N,N'-Dimethylformamid bzw. in 1,3-Dimethyl-imidazolidinon herstellbar sein soll. Wie sich beim Nacharbeiten der Beispiele der JP-B-4-8425 jedoch gezeigt hat, entstehen bei den beschriebenen Reaktionen in Wahrheit absolut keine N,N'-dicyclischen Harnstoffe.

Unerwarteterweise wurde nun gefunden, daß die freie Aminogruppe in einem Harnstoff, in dem eine der beiden Aminogruppen alkyliert ist, mit einer difunktionellen Alkylengruppe dialkyliert werden kann, wobei ein N-cyclischer Harnstoff gebildet wird, wenn man einen Phasentransferkatalysator verwendet, ohne daß dabei N,N'-Überbrückungen auftreten. Das neue Verfahren ist dabei nicht nur zur Herstellung der tetraalkylierten, N-cyclischen oder N,N'-dicyclischen Harnstoffe der Formel I, sondern auch zur Herstellung trialkylierter N-cyclischer Harnstoffderivate oder zur Herstellung N-cyclischer Diharnstoffe geeignet.

Gegenstand der Erfindung ist daher auch ein Verfahren zur Herstellung von Harnstoffen der allgemeinen Formel

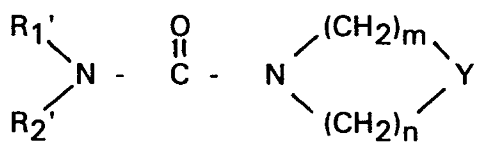

II

in der Y, m und n die in Anspruch 1 angegebenen Bedeutung haben und $R_1$' oder $R_2$' die in Anspruch 1 angegebenen Bedeutung von $R_1$ und $R_2$ haben und $R_1$'zusätzlich Wasserstoff bedeutet oder $R_1$' bedeutet Wasserstoff und $R_2$' eine Gruppe der Formel

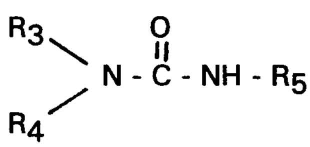

III

in der $R_3$ und $R_4$ die in Anspruch 1 angegebene Bedeutung von $R_1$ und $R_2$ haben, wobei $R_3$ und $R_4$ zusätzlich Wasserstoff und $R_5$ eine Alkylen- oder Alkylenarylenalkylengruppe bedeuten, das dadurch gekennzeichnet ist, daß ein Harnstoff oder ein Diharnstoff der allgemeinen Formel

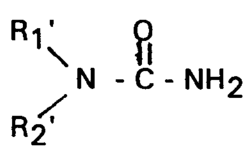

IV

in der $R_1$' und $R_2$' die obgenannte Bedeutung haben, in Gegenwart einer festen Base und eines Phasentransferkatalysators in einem unter den Reaktionsbedingungen inerten Verdünnungsmittel bei Temperaturen von 0 bis 150 °C mit einer Verbindung der allgemeinen Formel

$X - R_6 - X$ V

in der $R_6$ eine geradkettige Alkylengruppe mit 4 oder 5 C-Atomen, in der das Atom in der 2- oder 3-Position durch ein Sauerstoff- oder Schwefelatom ersetzt sein kann und X eine Halogen-, Sulfonsäure- oder Hydrogensulfatabgangsgruppe bedeutet, umgesetzt wird, wobei die $-NH_2$-Gruppe des Harnstoffes der allgemeinen Formel IV unter Abspaltung der beiden Wasserstoffatome durch die Verbindung der allgemeinen Formel V unter Abspaltung der Abgangsgruppen X unter Ringschluß dialkyliert wird.
In der Verbindung der allgemeinen Formel II haben $R_1'$ und $R_2'$ die oben angegebenen Bedeutungen für $R_1$ und $R_2$, wobei $R_1'$ zusätzlich Wasserstoff bedeutet, oder, falls die Verbindung die Formel II ein Diharnstoff ist, bedeutet $R_1'$ ein Wasserstoffatom und $R_2'$ eine Gruppe der allgemeinen Formel III.
In der Verbindung der allgemeinen Formel III haben $R_3$ und $R_4$ die in Anspruch 1 angegebene Bedeutung von $R_1$ und $R_2$, wobei $R_3$ und $R_4$ zusätzlich Wasserstoff und $R_5$ eine Alkylengruppe mit 2 bis 20, bevorzugt mit 2 bis 8 C-Atomen, beispielsweise Ethylen-, Hexylen-, Dodecylengruppen oder eine Alkylenarylenalkylengruppe, in der der Ausdruck Alkylen bevorzugt niedere Alkylengruppen mit 1 bis 3 C-Atomen und der Ausdruck Arylen bevorzugt eine Phenylengruppe, beispielsweise Xylylengruppen bedeuten.

Harnstoffe oder Diharnstoffe der allgemeinen Formel IV, in der $R_1'$ und $R_2'$ die oben angegebenen Bedeutung haben, sind nach einem der üblichen, bekannten Verfahren, etwa durch Umsetzung von Harnstoff oder Isocyansäure mit einem entsprechenden Amin herstellbar.

Als Basen kommen feste Basen, wie Alkalihydroxide, z.B. Kaliumhydroxid, Natriumhydroxid oder Alkaliamide, z.B. Natriumamid oder Kaliumamid in Frage. Bevorzugt werden Alkalihydroxide eingesetzt, wobei das Alkalihydroxid einen geringen Gehalt eines Carbonates wie Kaliumcarbonat, Natriumcarbonat, der 2 bis 20 Mol.% bezogen auf das Alkalihydroxid beträgt, aufweisen kann. Die Base wird in fester, gepulverter Form oder in Form von Pellets im Überschuß, bezogen auf den eingesetzten Harnstoff der Formel IV, verwendet. Bevorzugt werden pro Mol Harnstoff der Formel IV 1,5 bis 10 Mol, besonders bevorzugt 3 bis 5 Mol der festen Base eingesetzt.

Als Katalysator kommen übliche Phasentransferkatalysatoren in Frage. Eine Zusammenfassung von verwendbaren Phasentransferkatalysatoren und ihr möglicher Einsatz in verschiedenen Verdünnungsmitteln ist in W.E.Keller: Phasentransfer reactions (Fluka Compendium, Vol. 1, 2 und 3; Georg Thieme Verlag Stuttgart - New York, 1986, 1987 und 1992) geoffenbart. Bevorzugt werden als Phasentransferkatalysatoren quarternäre Ammoniumsalze, wie z.B. Tetrabutylammoniumhydrogensulfat, Tetrabutylammoniumchlorid oder Benzyltriethylammoniumchlorid eingesetzt.

In der Verbindung der allgemeinen Formel V bedeutet $R_6$ eine geradkettige Alkylengruppe mit 4 oder 5 C-Atomen, in der eines der C-Atome in der 2- oder 3-Position durch ein Sauerstoff- oder Schwefelatom, bevorzugt durch ein Sauerstoffatom ersetzt sein kann.

X steht für ein Halogenatom, wobei unter Halogen insbesonders Chlor, Brom oder Jod zu verstehen ist, eine Sulfonsäure- oder eine Hydrogensulfatgruppe, bevorzugt für ein Halogenatom.

Die Verbindungen der Formel V werden im allgemeinen äquimolar zum Harnstoff der Formel IV eingesetzt, sofern der Harnstoff der Formel IV nicht ein Diharnstoff ist oder sofern der Harnstoff der Formel IV ein Diharnstoff ist, in dem nur eine der beiden möglichen $-NH_2$-Gruppen dialkyliert werden soll. Sollen in einem Diharnstoff der Formel IV, in dem $R_3$ und $R_4$ Wasserstoff bedeuten, beide $-NH_2$-Gruppen alkyliert werden, werden im allgemeinen 2 Äquivalente einer Verbindung der Formel V pro Äquivalent Diharnstoff eingesetzt. Im Einzelfall kann aber ein Überschuß des einen oder anderen Reaktionspartners nützlich sein. So hat sich herausgestellt, daß in manchen Fällen die Ausbeute gesteigert werden kann, wenn 0,5 bis 3 Äquivalente der Verbindung der Formel V pro $-NH_2$-Gruppe im Harnstoff der Formel IV eingesetzt werden.

Als Verdünnungsmittel werden unter den Reaktionsbedingungen inerte Verdünnungsmittel, welche Lösungsmittel für den Harnstoff der Formel IV und/oder die Verbindung der Formel V sind, eingesetzt. Es sind dies aromatische Kohlenwasserstoffe, z.B. Benzol, Toluol, Xylole, höhere aliphatische Kohlenwasserstoffe, z.B. Paraffine, aromatische halogenierte Kohlenwasserstoffe z.B. Chlorbenzol, Trichlorbenzole, Ether, z.B. Tetrahydrofuran oder Dimethylsulfoxid oder Mischungen solcher Verdünnungsmittel. Bevorzugt werden aromatische Kohlenwasserstoffe, besonders bevorzugt wird Toluol eingesetzt.

Zur Durchführung des erfindungsgemäßen Verfahrens wird der Harnstoff der Formel IV in dem Verdünnungsmittel, das vor dem Einsatz vorgetrocknet werden kann, gelöst. Die feste Base wird in Form von Pellets oder in gepulverter Form zugesetzt und durch starkes Rühren gut suspendiert, worauf der Katalysator eingebracht wird. Die Verbindung der Formel V kann in diese Mischung, die stark gerührt und gegebenenfalls erhitzt wird, noch vor dem Erhitzen zugegeben werden oder sie wird in die erhitzte Mischung zugegeben.

Die Reaktionsmischung wird gegebenenfalls auf Temperaturen bis etwa 150 °C, bevorzugt auf 70 bis 150 °C, besonders bevorzugt auf Rückflußtemperatur des jeweils verwendeten Verdünnungsmittels erhitzt. Dabei wird die $-NH_2$-Gruppe des Harnstoffes der Formel IV durch die Verbindung der Formel V unter Abspaltung der beiden Abgangsgruppen X und der beiden Wasserstoffatome der $-NH_2$-Gruppe dialkyliert und es entsteht der Harnstoff der allgemeinen Formel II. Unerwarteterweise finden dabei praktisch keine N,N'-Überbrückungen oder Bindungen von 2 Molen des Harnstoffes der allgemeinen Formel IV aneinander statt.

Nach beendeter Umsetzung wird die Reaktionsmischung entweder gegebenenfalls erkalten gelassen und filtriert und der flüssige Rückstand destilliert oder chromatographiert, oder der Reaktionsmischung wird Wasser zugesetzt und der Harnstoff der Formel II mit Hilfe eines Extraktionsmittels aus der Reaktionsmischung extrahiert. Als Extraktionsmittel werden mit Wasser nicht mischbare, organische Extraktionsmittel, wie Kohlenwasserstoffe, beispielsweise Hexan, Heptan, halogenierte Kohlenwasserstoffe, beispielsweise Methylenchlorid, Chloroform oder Ether beispielsweise Diethylether, Diisopropylether, Carbonsäureester, wie Essigsäureethylester, Essigsäurebutylester eingesetzt. Die organische Phase wird mit Wasser gewaschen, getrocknet und das Verdünnungsmittel verdampft, wobei eine Nachtrocknung im Vakuum erfolgen kann.
Im allgemeinen ist die Reinheit des auf diese Weise hergestellten Harnstoffes der Formel II ausreichend. Gegenbenenfalls kann auch noch ein Reinigungsschritt, z.B. durch Chromatographie oder Destillation, angeschlossen werden.

In einer bevorzugten Ausführungsform wird ein Harnstoff der Formel IV, in der $R_1'$ und $R_2'$ unabhängig voneinander eine Alkylgruppe mit 1 bis 10 C-Atomen und $R_1'$ zusätzlich Wasserstoff oder $R_1'$ und $R_2'$ gemeinsam mit dem Stickstoffatom einen Pyrrolidin-, Piperidin- oder Morpholinring bedeuten, in Toluol gelöst, mit 3 bis 5 Äquivalenten Kalium- oder Natriumhydroxidpellets, die 4 bis 10 Mol% Kalium- oder Natriumkarbonat enthalten, sowie mit 0,04 bis 0,06 Äquivalenten eines quarternären Ammoniumsalzes als Phasentransferkatalysator unter starkem Rühren versetzt, auf Rückfluß erhitzt und mit 1,4-Dihalogenbutan oder 1,5-Dihalogenpentan, worin eines der C-Atome in 2- oder 3-Position durch ein Sauerstoffatom ersetzt sein kann, versetzt. Nach beendeter Reaktion wird der Reaktionsmischung Wasser zugegeben und mit Methylenchlorid und/oder Chloroform mehrmals extrahiert. Die vereinigten organischen Phasen werden mit Wasser gewaschen, getrocknet und das Verdünnungsmmittel verdampft, worauf im Vakuum nachgetrocknet wird.

Auf die beschriebene Art und Weise werden aus ungiftigen Ausgangsstoffen N-cyclische oder N,N'-dicyclische Harnstoffe in guten Ausbeuten und guter Reinheit hergestellt.

## Beispiel 1

3,56 g N-Piperidincarbonsäureamid (0,02 Mol) wurde in 40 ml Toluol gelöst, mit 4,48 g KOH (0,08 Mol), 0,28 g Tetrabutylammoniumchlorid (1 mMol) und mit 2,16 g 1,4-Dibrombutan (0,01 Mol) bei Raumtemperatur versetzt und unter starkem Rühren auf Rückfluß erhitzt. Der Verlauf der Reaktion wurde mit Hilfe von $^1$H-NMR verfolgt. Nach 2 Stunden war die Reaktion beendet und die Reaktionsmischung wurde in Wasser gegossen. Die wäßrige Mischung wurde mehrmals mit Methylenchlorid extrahiert, die organische Phase getrocknet und abgedampft. Dabei wurden 1,73 g, das sind 95 % der Theorie, 1-Piperidino-1-pyrrolidinocarbonyl, bezogen auf eingesetztes 1,4-Dibrombutan, erhalten.

$^1$H-NMR (300MHz, $CDCl_3$, delta): 3,35 ppm (t, Py-1,4; J = 5,6 Hz); 3,18 ppm (t, Pip-1,5; J = 6,7 HZ); 1,81 ppm (m; Py-2,3); 1,57 ppm (m; Pip-2,3,4)
$^{13}$C-NMR (70 MHz, $CDCl_3$, delta): 163,53 ppm (C = O); 48,38 ppm (Py-1,4); 47,45 ppm (Pip-1,5); 25,90 ppm (Pip-2,4); 25,57 ppm (Py-2,3); 24,57 ppm (Pip-3)

## Beispiel 2 - 11

Die folgenden Beispiele 2 bis 11 wurden in der in Beispiel 1 beschriebenen Art und Weise unter Verwendung derselben KOH- und Katalysatormenge pro Mol Harnstoff der Formel IV, aber unter Verwendung verschiedener Harnstoffe der Formel IV und unter Verwendung verschiedener Verbindungen der Formel V in verschiedenen Molverhältnissen durchgeführt. Die Ergebnisse sind in Tabelle 1 zusammengefaßt. Die Reaktionszeit betrug jeweils etwa 2 Stunden.

**Tabelle 1**

| Nr. | IV-$R_1$' | IV-$R_2$' | V-$R_6$ | V-X | IV: | VA% |
|---|---|---|---|---|---|---|
| 2 | $C_2H_5$- | $C_2H_5$ | $-(CH_2)_4-$ | Br | 2:1 | 65 |
| 3 | $C_2H_5$- | $C_2H_5$- | $-(CH_2)_4-$ | Br | 1:1 | 53 |
| 4 | $-(CH_2)_4-$ | | $-(CH_2)_4-$ | Br | 2:1 | 71 |
| 5 | $-(CH_2)_4-$ | | $-(CH_2)_5-$ | Br | 2:1 | 50 |
| 6 | $-(CH_2)_5-$ | | $-(CH_2)_4-$ | Cl | 2:1 | 73 |
| 7 | $-(CH_2)_5-$ | | $-(CH_2)_5-$ | Br | 2:1 | 61 |
| 8 | $-(CH_2)_2-O-(CH_2)_2-$ | | $-(CH_2)_4-$ | Br | 1:1 | 76 |
| 9 | $-(CH_2)_2-O-(CH_2)_2-$ | | $-(CH_2)_4-$ | Br | 2:1 | 80 |
| 10 | $-(CH_2)_2-O-(CH_2)_2-$ | | $-(CH_2)_5-$ | Br | 2:1 | 65 |
| 11 | H | $C_4H_9$- | $-(CH_2)_4-$ | Br | 2:1 | 67 |

**Charakterisierungsdaten:**

Beispiele 2 und 3

$^{1}$H-NMR (300MHz, $CDCl_3$, delta): 3,257 ppm (t, Py-1, $J_{CH2CH2}$ = 6,6 Hz); 3,126 ppm (q; Ethyl-1; $J_{CH2CH3}$ = 5,3 Hz); 1,742 ppm (m; Py-2,3); 1,048 ppm (t; Ethyl-2; $J_{CH2CH3}$ = 6,6 Hz)
$^{13}$C-NMR (70 MHz, $CDCl_3$, delta): 163,01 ppm (C = O); 48,68 ppm (Py-1,4); 42,00 ppm (Ethyl-1); 25,87 ppm (Ethyl-2); 13,78 ppm (Py-2,3)

Beispiel 4

$^{1}$H-NMR (300MHz, $CDCl_3$, delta): 3,36 ppm (t; N-$CH_2$; J = 5,5 Hz); 1,83 ppm (m; $CH_2$-$CH_2$)
$^{13}$C-NMR (70 MHz, $CDCl_3$, delta): 161,0 ppm (C = O); 47,7 ppm (N-$CH_2$); 25,1 ppm ($CH_2$-$CH_2$)

Beispiele 5 und 6

Charakterisierungsdaten wie in Beispiel 1 beschrieben.

Beispiel 7

$^{1}$H-NMR (300MHz, $CDCl_3$, delta): 3,16 ppm (t; Pip-1,5; J = 5,7); 1,57 ppm (m; Pip-2,3,4)
$^{13}$C-NMR (70 MHz, $CDCl_3$, delta): 164,75 ppm (C = O); 48,13 ppm (Pip-1,5); 25,71 ppm (Pip-2,4); 24,99 ppm (Pip-3)

Beispiele 8 und 9

$^{1}$H-NMR (300MHz, $CDCl_3$, delta): 3,67 ppm (t; Mor-O-$CH_2$; J = 4,7 Hz); 3,37 ppm (t; Pyr-N-$CH_2$; J = 6,7 Hz); 3,26 ppm (t; Mor-N-$CH_2$; J = 4,7 Hz); 1,84 ppm (m; Pyr-N-$CH_2$-$CH_2$)
$^{13}$C-NMR (70 MHz, $CDCl_3$, delta): 162,58 ppm (C = O); 66,71 ppm (Mor-O-$CH_2$); 48,25 ppm (Pyr-N-$CH_2$); 46,78 ppm (Mor-N-$CH_2$); 25,51 ppm (Pyr-2,3)

Beispiel 10

$^{1}$H-NMR (300MHz, $CDCl_3$, delta): 3,66 ppm (t; Mor-O-$CH_2$); J = 4,8 Hz); 3,22 ppm (m; Pip-1,5 und Mor-N-$CH_2$); 1,57 ppm (m; Pip-2,3,4)
$^{13}$C-NMR (70 MHz, $CDCl_3$, delta): 164,56 ppm (C = O); 67,00 ppm (Mor-O-$CH_2$); 48,33 ppm (Pip-N-$CH_2$);

47,86 ppm (Mor-N-$CH_2$); 26,10 ppm (Pip-2,4); 24,57 ppm (Pip-3)

Beispiel 11

$^1$H-NMR (200MHz, $CDCl_3$, delta): 4,390 ppm (t; NH; J = 5,8 Hz); 3,340 ppm (t; N-$CH_2$; $J_{12}$ = 6,7 Hz); 3,222 ppm (dt; HN-$CH_2$; $J_{CH2NH}$ = 5,8 Hz; $J_{CH2CH2}$ = 7,0 Hz); 1,893 ppm (tt; Pyr-2,3; $J_{12}$ = 6,7 Hz; $J_{23}$ = 3,5 Hz); 1,536-1,288 ppm (m; But-2,3); 0,921 ppm (t; But-$CH_3$; $J_{CH2CH3}$ = 7,1 Hz)
$^{13}$C-NMR (50 MHz, $CDCl_3$, delta): 156,016 ppm (C = O); 45,41 ppm (Pyr-1,4); 40,28 ppm (But-1); 32,61 ppm (But-2); 25,52 ppm (Pyr-2,3); 20,03 ppm (But-3); 13,78 ppm (But-4)

**Beispiel 12**

22,8 g Pyrrolidinocarbonsäureamid (0,2 Mol) wurden in 400 ml Toluol gelöst, mit 56 g KOH (0,8 Mol), 2,78 g Tetrabutylammoniumchlorid (1 mMol) und 21,6 g 1,4-Dibrombutan (0,1 Mol) bei Raumtemperatur versetzt und unter starktem Rühren auf Rückfluß erhitzt. Die Reaktion wurde mit Hilfe von $^1$H-NMR verfolgt. Nach beendeter Reaktion wurde das Lösungsmittel abgedampft und der Rückstand im Vakuum destilliert. Dabei wurden 8,27 g 1,1-Carbonylbispyrrolidin, das sind 54 % der Theorie, bezogen auf das eingesetzte 1,4-Dibrombutan, erhalten.

Charakterisierungsdaten wie im Beispiel 4 beschrieben.

**Beispiele 13 - 16**

Die Beispiele 13 bis 16 wurden in der im Beispiel 12 beschriebenen Art und Weise unter Verwendung derselben KOH- und Katalysatormenge durchgeführt, wobei aber im Beispiel 13 eine achtfach molare KOH-Menge bezogen auf die eingesetzte Verbindung der Formel V verwendet wurde und wobei im Beispiel 14 die Verbindung der Formel V erst nach Erhitzen des Gemisches aus Harnstoff der Formel I, Toluol, Base und Katalysator auf Rückfluß zugetropft wurde. Die Reaktionszeiten betrugen jeweils etwa 2 Stunden mit Ausnahme von Beispiel 15. In Beispiel 15 betrug die Reaktionszeit 0,15 Stunden. Die Ergebnisse sind in Tabelle 2 zusammengefaßt.

Tabelle 2

| Nr. | IV-$R_1$'-$R_2$' | V-$R_6$ | V-X | IV:V | A% |
|---|---|---|---|---|---|
| 13 | $-(CH_2)_4-$ | $-(CH_2)_4-$ | Br | 1:1 | 65 |
| 14 | $-(CH_2)_4-$ | $-(CH_2)_4-$ | Br | 1:1 | 59 |
| 15 | $-(CH_2)_4-$ | $-(CH_2)_4-$ | Br | 1:1 | 67 |
| 16 | $-(CH_2)_4-$ | $-(CH_2)_4-$ | Br | 1:3 | 72 |

Charkaterisierungsdaten wie im Beispiel 4 beschrieben.

In den Tabellen 1 und 2 bedeuten:

Nr.: Nummer des Beispiels und der Verbindung
IV-$R_1$': $R_1$' in der Formel IV
IV-$R_2$': $R_2$' in der Formel IV
V-$R_6$: $R_6$ in der Formel V
V-X: X in der Formel V
IV:V: Molverhältnis der Verbindungen der Formeln IV und V.
A%: Ausbeute in Molprozent jeweils bezogen auf die eingesetzte Verbindung der Formel V. Nur in Beispiel 16 ist die Ausbeute auf die eingesetzte Verbindung der Formel IV bezogen.

**Vergleichsbeispiele**

a) Beispiel 7 aus JP-B-4-8425

13 g Harnstoff in 150 ml N,N'-Dimethylformamid wurden mit 96 g 1,4-Dibrombutan und 50 g Kaliumhydroxid 4 Stunden bei einer Temperatur von 20 °C gerührt. Danach wurden die nichtlöslichen Teile abfiltriert und das Filtrat destilliert, wobei 4 Fraktionen zwischen 23 und 80 °C bei 0,4 mm Hg abgenommen wurden. Die Fraktionen wurden mit Hilfe von $^1$H-NMR Spektroskopie analysiert. Dabei wurde nur 1,4-Dibrombutan und N,N'-Dimethylformamid, aber absolut kein Carbonylbispyrrolidin gefunden.

b) Beispiel 8 aus JP-B-4-8425

13 g Harnstoff in 150 ml 1,3-Dimethyl-2-imidazolidinon wurden mit 150 g 1,5-Dibrompentan und 50 g Kaliumhydroxid 4 Stunden bei einer Temperatur von 20 °C gerührt. Danach wurden die ungelösten Teile abfiltriert und das Filtrat destilliert. Die Fraktion von 23 bis 76 °C bei 0,35 mm Hg wurde abgenommen und mit Hilfe der $^{1}$H-NMR Spektroskopie analysiert. Dabei wurde nur 1,5-Dibrompentan und 1,3-Dimethyl-2-imidazolidinon, aber absolut kein Carbonylbispiperidin gefunden.

## Patentansprüche

**1.** Verwendung eines Harnstoffes der allgemeinen Formel

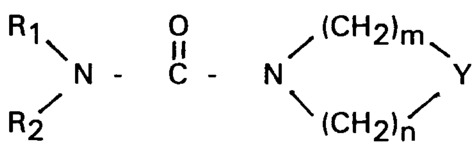

I

in der $R_1$ und $R_2$ unabhängig voneinander geradkettige, verzweigte oder cyclische, unsubstituierte oder durch Fluoratome, Nitrogruppen, Alkenyl-, Alkyliden-, Aryl-, Alkoxy- oder Phenoxygruppen substituierte Alkyl- oder Aralkylgruppen oder gemeinsam mit dem Stickstoffatom einen 5- oder 6-gliedrigen, nicht aromatischen Ring, der durch ein Sauerstoff- oder Schwefelatom durchbrochen sein kann, Y eine Methylengruppe, ein Sauerstoff- oder Schwefelatom und n und m unabhängig voneinander die Zahlen 1 bis 3, wobei n plus m die Zahlen 3 oder 4 sind, bedeuten, als chemisches Lösungsmittel.

**2.** Verwendung nach Anspruch 1, dadurch gekennzeichnet, daß $R_1$ und $R_2$ unabhängig voneinander geradkettige, unsubstituierte Alkylgruppen oder gemeinsam mit dem Stickstoffatom einen unsubstituierten 5- oder 6-gliedrigen, nicht aromatischen Ring, der durch ein Sauerstoffatom durchbrochen sein kann, bedeuten.

**3.** Verwendung nach einem der Ansprüche 1 oder 2, dadurch gekennzeichnet, daß Y eine Methylengruppe oder einen Sauerstoffatom und n und m unabhängig voneinander die Zahlen 1 oder 2 und n plus m die Zahlen 3 oder 4 bedeuten.

**4.** Verwendung nach einem der Ansprüche 1 bis 3, dadurch gekennzeichnet, daß $R_1$ und $R_2$ Alkylgruppen mit 1 bis 6 C-Atomen oder gemeinsam mit dem Stickstoffatom einen 5- oder 6-gliedrigen, nicht aromatischen Ring, der durch ein Sauerstoffatom durchbrochen sein kann, bedeuten.

**5.** Verfahren zur Herstellung von Harnstoffen der allgemeinen Formel

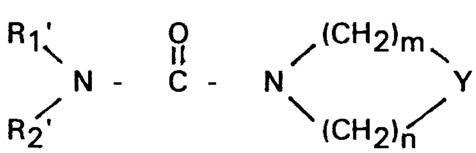

II

in der Y, m und n die in Anspruch 1 angegebene Bedeutung haben und $R_1$' oder $R_2$' die in Anspruch 1 angegebenen Bedeutung von $R_1$ und $R_2$ haben und $R_1$' zusätzlich Wasserstoff bedeutet oder $R_1$' bedeutet Wasserstoff und $R_2$' eine Gruppe der allgemeinen Formel

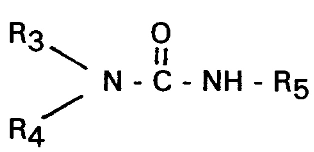

III

in der $R_3$ und $R_4$ die in Anspruch 1 angegebene Bedeutung von $R_1$ und $R_2$ haben, wobei $R_3$ und $R_4$

zusätzlich Wasserstoff und $R_5$ eine Alkylen- oder Alkylenarylenalkylengruppe bedeuten, dadurch gekennzeichnet, daß ein Harnstoff oder ein Diharnstoff der allgemeinen Formel

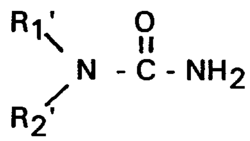

IV

in der $R_1'$ und $R_2'$ die obgenannte Bedeutung haben, in Gegenwart einer festen Base und eines Phasentransferkatalysators in einem unter den Reaktionsbedingungen inerten Verdünnungsmittel bei Temperaturen von 0 bis 150 °C mit einer Verbindung der allgemeinen Formel

$X - R_6 - X$ V

in der $R_6$ eine geradkettige Alkylengruppe mit 4 oder 5 C-Atomen, in der das C-Atom in der 2- oder 3-Position durch ein Sauerstoff- oder Schwefelatom ersetzt sein kann und X eine Halogen-, Sulfonsäure- oder Hydrogensulfatabgangsgruppe bedeutet, umgesetzt wird, wobei die $-NH_2$ Gruppe des Harnstoffes der allgemeinen Formel IV unter Abspaltung der beiden Wasserstoffatome durch die Verbindung der allgemeinen Formel V unter Abspaltung der Abgangsgruppen X unter Ringschluß dialkyliert wird.

**6.** Verfahren nach Anspruch 5, dadurch gekennzeichnet, daß ein Harnstoff der allgemeinen Formel IV, in der $R_1'$ und $R_2'$ unabhängig voneinander eine geradkettige Alkylgruppe mit 1 bis 8 C-Atomen und $R_1'$ zusätzlich Wasserstoff oder $R_1'$ und $R_2'$ gemeinsam mit dem Stickstoffatom ein 5- oder 6-gliedrigen, nicht aromatischen Ring, der durch ein Sauerstoffatom durchbrochen sein kann, bedeuten, eingesetzt wird.

**7.** Verfahren nach einem der Ansprüche 5 oder 6, dadurch gekennzeichnet, daß eine Verbindung der allgemeinen Formel V, in der X ein Halogenatom bedeutet, eingesetzt wird.

**8.** Verfahren nach einem der Ansprüche 5 bis 7, dadurch gekennzeichnet, daß als Verdünnungsmittel ein aromatischer Kohlenwasserstoff eingesetzt wird.

**9.** Verfahren nach einem der Ansprüche 5 bis 8, dadurch gekennzeichnet, daß als Base Kalium- oder Natriumhydroxid und als Phasentransferkatalysator ein quarternäres Ammoniumsalz eingesetzt wird.

Europäisches Patentamt

# EUROPÄISCHER RECHERCHENBERICHT

Nummer der Anmeldung
EP 94 10 0394

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | US-A-2 868 328 (MONSANTO CHEMICAL COMPANY) *Insgesamt* | 1 | C07D295/20 |
| A | CH-A-367 171 (CILAG-CHEMIE AKTIENGESELLSCHAFT) *Insgesamt* | | |
| A | EP-A-0 467 045 (BAYER) *Insgesamt* | | |
| D,A | CHEMICAL ABSTRACTS, vol. 112, no. 21, 21. Mai 1990, Columbus, Ohio, US; abstract no. 198399j, Seite 719 ;Spalte L ; * Zusammenfassung * & JP-A-01 287 078 (MITSUI TOATSU CHEMICALS) | 5-9 | |
| D,A | METHODEN DER ORGANISCHEN CHEMIE(HOUBEN-WEYL) Bd. E4 , 1983 , STUTTGART Seite 335 | 1 | |
| | | | RECHERCHIERTE SACHGEBIETE (Int.Cl.5) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 25. April 1994 | Luyten, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)